# EUROPEAN PATENT APPLICATION

(11) **EP 4 763 147 A1**
(43) Date of publication of application: **24.06.2026**
(21) Application number: 24855666.4
(22) Date of filing: 14.08.2024
(51) Int. Cl.: A61F 2/844, A61F 2/90, A61B 17/00

(54) **IMPLANT AND IMPLANT SYSTEM**

(30) Priority: 18.08.2023 CN 202311051325
(71) Applicant: United Innomed (Shanghai) Limited, Shanghai 201315 (CN)
(72) Inventor: GE, Shuchen, Shanghai 201315 (CN); LI, Bo, Shanghai 201315 (CN); WANG, Li, Shanghai 201315 (CN)
(74) Representative: Murgitroyd & Company
(86) International application number: PCT/CN2024/111907
(87) International publication number: WO 2025/039940

(57) **Abstract**

An implant and an implant system. The implant is configured for being implanted into a target site in a living body. The implant includes a stent (100). The stent (100) is configured to be disposed at the target site to improve a physiological function of the living body. The stent (100) includes a tubular main body (110) made of a metallic material. A circumferential surface of the tubular main body (110) is provided with an opening (130), and the opening (130) extends over at least a portion of a length of the tubular main body (110). The implant facilitates smooth transmission of wireless signals.

## Description

### TECHNICAL FIELD

The present invention relates to the field of medical apparatus, and in particular, to an implant and an implant system.

### BACKGROUND

Heart failure is a clinical syndrome caused by abnormalities in ventricular filling and ejection function. Heart failure is mainly manifested as dyspnea, fatigue, fluid retention (pulmonary congestion, systemic circulation congestion, and peripheral edema), and the like. Heart failure may be classified into three categories, namely heart failure with reduced ejection fraction, heart failure with mildly ejection fraction, and heart failure with preserved ejection fraction. In patients with chronic heart failure, whether with preserved or reduced ejection fraction, sustained elevation of left atrial pressure is a primary cause of pulmonary congestion, accounting for 90% of hospitalizations among heart failure patients.

An atrial shunt is a therapeutic approach to improve elevated left atrial pressure by creating an aperture in the atrial septumatrial septum to shunt blood flow from the left atrium to the right atrium, thereby reducing a load on the left atrium. Atrial shunt is categorized into implant-based shunting and non-implant shunting. Implant-based shunting requires the implant to be fixed in an artificially formed atrial septum aperture to keep the aperture unclosed. Currently, atrial septum aperture creation and implant fixation are generally performed by means of vascular intervention. Specifically, an apparatus or a shunt is accommodated in a catheter, and then the apparatus or the shunt is operated with the catheter through a human artery or a vein into a heart lesion site for operation. Due to a limited diameter of the blood vessel, a corresponding catheter diameter is also limited. Therefore, the shunt needs to first maintain in a compressed state in the catheter, and then automatically or passively expand to a predetermined size after being removed from the catheter when the shunt is delivered to a target site.

In addition to a shunt suitable for patients with heart failure described above, for patients with cardiovascular diseases, there are other stent-like implants suitable for placement within the heart or blood vessels. Such as some patient may require an occluder in the heart or blood vessels to improve cardiovascular function, while patients with vascular stenosis may need to be implanted with a vascular stent. However, in addition to treating related symptoms by means of an implant, patients may also need to implant with other components for wireless communication with the outside; for example, a wireless monitoring device for monitoring hemodynamic parameters of the patient's cardiovascular system. For heart failure patients, in addition to surgical treatment, monitoring a pressure of the atrium is also an important health management means. Therefore, under specific conditions, a wireless pressure monitoring device may be placed on the atrial septum. Nevertheless, because stents are typically mesh structures formed by weaving metal wires or by cutting a tubular material, the mesh structure may interfere with or even shield wireless signal transmission of wireless communication components. On the other hand, if the pressure monitoring device is placed axially at a proximal or distal end of the stent, it would increase an overall structural length, hinder normal blood flow, affect a size of a stent end, and create a risk of long-term stenosis.

### SUMMARY

In view of the above, the present invention provides an implant and an implant system, which are used to solve at least some of the problems mentioned above.

In a first aspect, the present invention provides an implant for implanting at a target site within a living body. The implant includes a stent. The stent is configured to be disposed at the target site to improve a physiological function of the living body. The stent includes a tubular main body made of a metallic material, a circumferential surface of the tubular main body is provided with an opening, and the opening extends over at least a portion of a length of the tubular main body.

In one possible implementation, the opening includes at least a first open end.

In combination with the above possible implementations, in one possible implementation, the opening is distributed over an entire length of the tubular main body and further has a second open end.

In combination with the above possible implementations, in one possible implementation, the opening extends on the circumferential surface of the tubular main body in a helical, linear, or wavy form.

In combination with the above possible implementations, in one possible implementation, when the opening extends on the circumferential surface of the tubular main body in a helical form, the opening extends for at least two turns around the circumferential surface of the tubular main body.

In combination with the above possible implementations, in one possible implementation, an elastic curved segment is helically coiled to form the tubular main body, and a gap between two adjacent turns of the elastic curved segment forms the opening.

In combination with the above possible implementations, in one possible implementation, the elastic curved segment is a metal wire or a metal strip cut from a metal member.

In combination with the above possible implementations, in one possible implementation, the elastic curved segment, starting from an initial position, extends circumferentially in an annular form for one turn, then extends helically upward for one or more turns, and then further extends in an annular form for one turn, thereby forming the tubular main body.

In combination with the above possible implementations, in one possible implementation, the elastic curved segment is a wave metal segment.

In combination with the above possible implementations, in one possible implementation, the opening divides the tubular main body into an open-loop structure having a C-shaped axial profile projection plane, and the open-loop structure has a first end portion and a second end portion opposite to each other.

In combination with the above possible implementations, in one possible implementation, the first end portion is not in contact with the second end portion, or is offset from the second end portion circumferentially or radially.

In combination with the above possible implementations, in one possible implementation, the tubular main body has a mesh structure, and the mesh structure is formed by weaving metal wires or by cutting a metal member.

In combination with the above possible implementations, in one possible implementation, when the open-loop structure is compressed, a layered structure having a helical axial profile projection plane is formed, and the first end portion is wound from an inner side of the second end portion toward a center.

In combination with the above possible implementations, in one possible implementation, the impant further includes a wireless sensor. The wireless sensor includes a detection element and a wireless signal transmission element, the detection element is configured to obtain a target physiological parameter, and the wireless signal transmission element is configured to transmit the target physiological parameter obtained by the detection element to an outside of the living body.

In combination with the above possible implementations, in one possible implementation, in a working state, the wireless sensor is located outside the tubular main body, and when the tubular main body is compressed, the wireless sensor is located side by side outside the tubular main body.

In combination with the above possible implementations, in one possible implementation, in a working state, the wireless sensor is located inside the tubular main body, and when the tubular main body is compressed, the wireless sensor is located inside the tubular main body.

In combination with the above possible implementations, in one possible implementation, the wireless sensor further includes a housing, and the detection element is located at one end of the housing and protrudes a predetermined distance relative to the tubular main body along a length direction of the housing.

In combination with the above possible implementations, in one possible implementation, the stent further includes a clamping arm assembly. The clamping arm assembly at least includes a first clamping arm and a second clamping arm, the first clamping arm and the second clamping arm are distributed on an outer periphery of the tubular main body at intervals along an axial direction of the tubular main body, and the first clamping arm and the second clamping arm each forms a cantilever protruding from the outer periphery of the tubular main body.

In combination with the above possible implementations, in one possible implementation, the first clamping arm and/or the second clamping arm are formed by bending an elastic metal wire or metal strip. The first clamping arm and/or the second clamping arm have a U-shaped or V-shaped structure formed by an elastic metal wire or metal strip extending radially a predetermined distance and then folding back.

In combination with the above possible implementations, in one possible implementation, the first clamping arm and/or the second clamping arm are integrally formed with the tubular main body.

In combination with the above possible implementations, in one possible implementation, the stent further includes a covering membrane. The covering membrane is applied to an inner side and/or an outer side of the tubular main body, and the covering membrane is made of an insulating material.

In combination with the above possible implementations, in one possible implementation, the implant further includes a wireless sensor and a connecting member. The stent and the wireless sensor are arranged in parallel, the wireless sensor is connected to an engaging segment of the stent via the connecting member, and the engaging segment is integrally formed with the tubular main body.

In combination with the above possible implementations, in one possible implementation, the engaging segment includes a first engaging segment and a second engaging segment. The connecting member includes a first connecting portion and a second connecting portion. The first connecting portion is connected to the wireless sensor, the second connecting portion includes a sheet portion, and the sheet portion is clamped by the first engaging segment and the second engaging segment.

In combination with the above possible implementations, in one possible implementation, a free end of the first engaging segment and a free end of the second engaging segment point in opposite directions.

In a second aspect, the present invention provides an implant system, which includes a delivery catheter and an implant. The implant is the implant according to any one of the implementations of the first aspect, the implant is in a compressed state when disposed within the catheter, and the implant is capable of protruding from the catheter.

In one possible implementation, the tubular main body is compressed into a structure having an axial profile projection plane that is C-shaped, helical, or wavy closed-loop.

In one possible implementation, when the tubular main body is compressed into the structure having the axial profile projection plane that is C-shaped, an outer surface of a compressed tubular main body at least partially surrounds the wireless sensor.

In one possible implementation, when the tubular main body is compressed into the structure having the axial profile projection plane that is helical, the wireless sensor is located side by side outside the tubular main body, or the wireless sensor is located inside the tubular main body.

In one possible implementation, when the tubular main body is compressed into the structure having the axial profile projection plane that is wavy closed-loop, the wireless sensor is located inside the tubular main body.

In combination with the above possible implementations, in one possible implementation, The implant system further includes a signal receiving device. The signal receiving device is configured to transmit signals with the wireless sensor in the implant.

In the implant provided by the present invention, the stent of the implant includes a tubular main body made of a metallic material. The opening provided on the circumferential surface of the tubular main body has at least a first open end. The first open end ensures that a solid structure enclosing the opening does not form a closed-loop structure in the circumferential direction within at least one axial region, thereby reducing wireless signal interference caused by the tubular main body made of the metallic material.

### BRIEF DESCRIPTION OF THE DRAWINGS

In order to more clearly illustrate technical solutions of embodiments of the present invention, the drawings to be used in the embodiments will be introduced briefly below.

It should be understood that, the drawings below merely show some embodiments of the present invention, and should not be construed as limiting the scope.

It should also be understood that, the same or similar reference numerals are used in the drawings for representing the same or similar elements.

It should also be understood that, the drawings are merely illustrative, and dimensions and proportions of the elements in the drawings are not necessarily accurate.
FIG. 1 is a schematic structural diagram of a target site m1 in a living body according to an embodiment of the present invention.
FIG. 2 is a schematic structural diagram of an implant according to an embodiment of the present invention.
FIG. 3 is a schematic structural diagram of the implant in FIG. 2 with a covering membrane omitted.
FIG. 4 is a schematic structural diagram of an elastic curved segment of a tubular main body of the implant in FIG. 2.
FIG. 5 is a schematic structural diagram of the implant in FIG. 2 with a wireless sensor omitted.
FIG. 6 is an enlarged schematic diagram of a junction between an upper segment and a lower segment of a tubular main body with a wireless sensor omitted.
FIG. 7 is a schematic diagram showing a compressed state of the implant according to the aforementioned embodiment when located within a delivery catheter.
FIG. 8 is a schematic diagram showing an axial profile projection plane of the delivery catheter and the implant in FIG. 7.
FIG. 9 is a schematic diagram showing a delivery catheter containing an implant upon reaching a target site.
FIG. 10 is a schematic diagram showing an implant with one end and a first clamping arm protruding from a delivery catheter.
FIG. 11 is a schematic diagram showing a first clamping arm of an implant reaching a surface of an atrial septum upon retraction of a delivery catheter.
FIG. 12 is a schematic diagram showing an implant being installed at a target site after withdrawal of a delivery catheter.
FIG. 13 is a schematic structural diagram of an implant according to another embodiment of the present invention.
FIG. 14 is a schematic structural diagram of an axial profile projection plane of the implant in FIG. 13 when located within a delivery catheter.
FIG. 15 is a schematic structural diagram of an implant according to another embodiment of the present invention.
FIG. 16 is a schematic structural diagram of an axial profile projection plane of the implant in FIG. 15 when located within a delivery catheter.
FIG. 17 is a schematic structural diagram of an implant according to another embodiment of the present invention.
FIG. 18 is a schematic structural diagram of a stent of the implant in FIG. 17 with a wireless sensor omitted.
FIG. 19 is a schematic structural diagram of an axial profile projection plane of the stent in FIG. 18 in a first state.
FIG. 20 is a schematic structural diagram of an axial profile projection plane of the stent in FIG. 18 in a second state.
FIG. 21 is a schematic structural diagram of an axial profile projection plane of the implant in FIG. 17 when located within a delivery catheter.
FIG. 22 is a schematic structural diagram of an axial profile projection plane of an implant when located within a delivery catheter in an optional embodiment.
FIG. 23 is a schematic structural diagram of a tubular main body of an implant according to another embodiment of the present invention.
FIG. 24a is a schematic diagram of a shape of a longitudinal profile projection plane of a tubular main body in an optional implementation.
FIG. 24b is a schematic diagram of a shape of a longitudinal profile projection plane of a tubular main body in another optional implementation.
FIG. 24c is a schematic diagram of a shape of a longitudinal profile projection plane of a tubular main body in another optional implementation.
FIG. 24d is a schematic diagram of a shape of a longitudinal profile projection plane of a tubular main body in another optional implementation.
FIG. 24e is a schematic diagram of a shape of a longitudinal profile projection plane of a tubular main body in another optional implementation.

### DETAILED DESCRIPTIONS OF THE EMBODIMENTS

The following describes embodiments of the present invention exemplarily with reference to the drawings. It should be understood that there may be many implementations of the present invention, and should not be construed as limited to the embodiments described herein. The embodiments described herein are merely for a more thorough and complete understanding of the present invention.

It should be understood that the term "including" and its variations used in the present invention are open inclusions, that is, "including but not limited to". The term "according to" means "at least in part according to", and the term "a plurality of" means "two and more than two."

It should be understood that, although terms such as "first" or "second" are used in the present invention for describing various elements, these elements are not limited by these terms, and these terms are merely used for distinguishing one element from another.

The present invention provides an implant for implanting at a target site within a living body to improve a physiological function of the living body and to obtain a target physiological parameter. The implant includes a stent, which specifically may be a mesh structure such as a shunt, an occluder, or a vascular stent. The stent is used to be disposed at the target site. The stent includes a tubular main body made of a metallic material. A circumferential surface of the tubular main body is provided with an opening. The opening extends over at least a portion of a length of the tubular main body and has at least a first open end. The opening provided on the circumferential surface of the tubular main body has at least the first open end. The first open end ensures that a solid structure of the stent forming the opening has no closed-loop metal structure at any circumferential position, thereby reducing wireless signal interference caused by the metallic tubular main body.

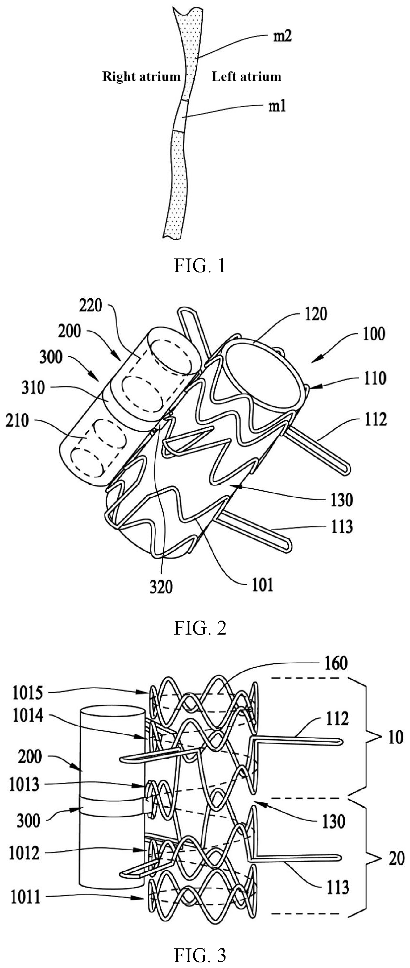

FIG. 1 is a schematic structural diagram of a target site m1 within a living body according to an embodiment of the present invention. Specifically, the target site m1 is an aperture in an atrial septum m2 located between a left atrium and a right atrium. The aperture may be an unclosed foramen ovale or an artificially created aperture. For some patients with heart failure, the aperture may reduce left atrial pressure and alleviate heart failure symptoms. The stent provided in the embodiment of the present invention is a shunt. The shunt may be disposed in the aperture to maintain a shape of the aperture and guide blood flow from the left atrium to the right atrium.

In this embodiment, the implant may further include a wireless sensor. The wireless sensor includes a detection element and a wireless signal transmission element electrically connected to each other. The detection element is configured to obtain a target physiological parameter, and the wireless signal transmission element is configured to transmit the target physiological parameter obtained by the detection element to an outside of the living body. At least a portion of the wireless sensor may be disposed within the tubular main body or outside the tubular main body, and wireless signals can be transmited in either configuration.

Preferably, the wireless sensor is disposed at a middle position of an outer circumferential surface of the shunt. Therefore, when the implant in this embodiment is disposed on the atrial septum m2, the wireless sensor and the stent are disposed within the target site m1 and abut against the target site. Further, two ends of the wireless sensor may be provided with two detection elements, enabling simultaneous acquisition of pressure data from the left atrium and the right atrium, or only one detection element may be disposed at one end of the wireless sensor to acquire pressure data solely from the left atrium.

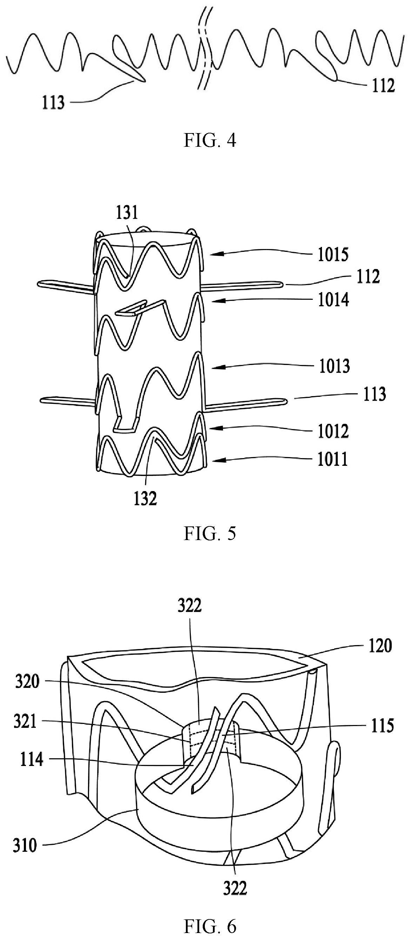

FIGS. 2 to 5 are schematic structural diagrams of an implant according to an embodiment of the present invention. Specifically, FIG. 2 is a schematic structural diagram of an implant according to an embodiment of the present invention. FIG. 3 is a schematic structural diagram of the implant in FIG. 2 with a covering membrane 120 omitted. FIG. 4 is a schematic structural diagram of an elastic curved segment 101 of a tubular main body 110 of the implant in FIG. 2. FIG. 5 is a schematic structural diagram of the implant in FIG. 2 with a wireless sensor 200 omitted.

The implant includes a stent 100, the wireless sensor 200, and a connecting member 300. The stent 100 and the wireless sensor 200 are arranged side by side and integrally connected via the connecting member 300. It should be understood that the term "side by side" in the present invention is not limited to a parallel arrangement. The stent 100 and the wireless sensor may also be positioned at a predetermined angle to each other (excluding a perpendicular relationship). The term "side by side" in the present invention is also not limited to outer surfaces of the two abutting against each other. It may also include a configuration in which the wireless sensor is disposed within the stent 100, with the outer surface of the wireless sensor abutting against an inner surface of the stent 100. Of course, preferably, the stent 100 and the wireless sensor 200 are arranged in parallel, and the wireless sensor 200 is connected to an engaging segment of the stent via the connecting member. Preferably, the engaging segment is integrally formed with the elastic curved segment. When a main body of the stent is a tubular structure and the wireless sensor 200 is also a tubular structure, under an influence of the connecting member and the engaging segment of the stent, the wireless sensor 200 and the stent 100 exhibit an externally tangent configuration. Such a design, relative to other manners of disposing the sensor 200 outside the stent 100, allows the implant to have a smaller radial dimension during a delivery process, facilitating its crimping within the delivery device. Furthermore, compared with a manner of placing the sensor 200 inside the stent 100, it can avoid interference by the sensor 200 with blood flow inside the stent 100.

In this embodiment, the stent 100 includes a tubular main body 110, a first clamping arm 112, a second clamping arm 113, and the covering membrane 120. The tubular main body 110 is a tubular structure formed by helically coiling an elastic curved segment 101, substantially cylindrical in shape. The elastic curved segment 101 is a metal wire. The elastic curved segment 101 is coiled in multiple turns in a helical form to form a hollow tubular structure. In this embodiment, the elastic curved segment 101 is coiled in five turns along a helical winding path 160, forming a first coil 1011, a second coil 1012, a third coil 1013, a fourth coil 1014, and a fifth coil 1015. An opening 130 is formed between two adjacent coils to connect an inside and outside of the tubular structure in a radial direction. In other words, the opening 130 is an irregular strip-shaped hollow portion extending helically on a circumferential surface of the tubular structure, and along this extending direction, two ends of the hollow portion are a first open end 131 and a second open end 132, respectively. Thus, the opening 130 is distributed over an entire length of the tubular main body 110, and both ends of the opening 130 remain unclosed, so that no closed metallic loop structure exists within an entire tubular main body. In some alternative embodiments, the opening 130 may be closed at one end, for example, an end of a metal wire at the first open end 131 contacts the fifth coil 1015 to form a closure. Since the opening 130 prevents a formation of any closed metallic loop structure in the entire tubular main body, signal transmission of the wireless sensor 200 is not affected by the tubular main body 110, regardless of whether the wireless sensor 200 is placed inside or outside the tubular main body 110. Compared to the prior art, when the wireless sensor 200 is placed inside the tubular main body 110, signal transmission performance may be significantly improved. When the wireless sensor 200 is placed outside the tubular main body 110, interference from the tubular main body 110 with signal transmission may also be reduced.

The first clamping arm 112 and the second clamping arm 113 extend radially outward from a surface of the tubular main body 110 to form cantilever structures. The first clamping arm 112 and the second clamping arm 113 form at least one clamping arm assembly, the two are configured to be positioned on two sides of the atrial septum m2 to clamp the atrial septum m2. Preferably, the tubular main body 110, the first clamping arm 112, and the second clamping arm 113 are all formed by winding and bending the elastic curved segment 101.

The tubular main body 110 is divided into an upper tubular segment 10 and a lower tubular segment 20. Both the first clamping arm 112 and the second clamping arm 113 are provided in a quantity of three. All three first clamping arm 112 are distributed on a surface of the upper tubular segment 10. All three second clamping arm 113 are distributed on a surface of the lower tubular segment 20. A portion of the elastic curved segment 101 extends in a wavy pattern along an axial direction while simultaneously undergoing a helical extension in a circumferential direction as a whole to form a mesh-like tubular body, and another portion of the elastic curved segment 101 extends radially outward from a surface of the mesh-like tubular body and then folds back onto the surface of the tubular body to form a U-shaped clamping arm. Specifically, while forming the mesh-like tubular body through the helical extension in the circumferential direction, the elastic curved segment 101 also extends radially outward from the surface of the mesh-like tubular body and then folds back onto the surface of the tubular body, thereby forming a substantially U-shaped clamping arm.

In other embodiments, the opening 130 may exist only in a section of the tubular main body, for example, in the upper tubular segment 10 of the tubular main body, while the wireless sensor 200 is disposed inside or outside the upper tubular segment 10. The tubular main body 110 may also include a plurality of elastic curved segments 101 that are not directly connected to each other, and relative positions of the elastic curved segments 101 may be maintained by the covering membrane 120, thereby achieving indirect connection. The tubular main body 110 may also be formed by cutting a metal member, and the elastic curved segment 101 and/or the clamping arm assembly are metal strips cut from the metal member. The metal member may be a metal tube or a metal sheet. When the metal member is a metal sheet, the tubular main body 110 is formed by cutting and then winding the metal sheet, with the elastic curved segment and the clamping arm assembly being integrally formed.

Furthermore, the three first clamping arms 112 and the three second clamping arms 113 form three clamping arm assemblies. In each clamping arm assembly, the first clamping arm 112 and the second clamping arm 113 are spaced apart in the axial direction. The first clamping arm 112 is positioned at a first predetermined distance from a first end (an upper end in FIG. 2) of the tubular main body 110. The second clamping arm 113 has a second predetermined distance from a second end (a lower end in FIG. 2) of the tubular main body 110. A free end of the first clamping arm 112 and a free end of the second clamping arm 113 overlap in an axial projection, while a fixed end of the first clamping arm 112 and a fixed end of the second clamping arm 113 are offset in the axial projection, thereby achieving a larger clamping area (an area of a clamping region formed jointly by the first clamping arm 112 and the second clamping arm 113), and obtaining a better clamping effect. Here, the fixed end of the first clamping arm 112 and the fixed end of the second clamping arm 113 refer to ends that are respectively connected to the tubular main body 110. The upper tubular segment 10 and the lower tubular segment 20 are arranged coaxially and the upper tubular segment 10 and the lower tubular segment 20 may be connected to each other via the covering membrane 120 and/or the elastic curved segment. The elastic curved segment of the lower tubular segment 20, starting from an initial position, extends circumferentially in an annular form for one turn and then extends upward in a helical form. The elastic curved segment of the upper tubular segment 10, starting from an initial position, extends upward in a helical form, with its final turn extending in an annular form. In the above embodiment, an axial distance between an upper and the lower clamping arm is 3 mm. An selectable range for the axial distance is greater than 0 mm and less than or equal to 5 mm. In the above embodiment, an elastic metal wire forming the clamping arms and the elastic curved segment of the tubular main body to which a base of the clamping arms is attached are the same elastic metal wire or metal strip. In some alternative implementations, the clamping arms may be separate U-shaped or V-shaped components, which is welded onto the elastic curved segment of the tubular main body by welding. The elastic curved segment of the upper tubular segment 10 and the elastic curved segment of the lower tubular segment 20 are continuous.

A design of the clamping arms in this embodiment differs from an anchor designs with waist-shaped or flange structures in the prior art. It can effectively reduce a volume of the shunt within a cavity and a contact area with anchor points, thus avoiding adverse effects on blood flow, and at a same time, stably anchor the main body at the target site.

The covering membrane 120 is applied to an inner surface of the tubular main body 110. In the embodiment, a length of the covering membrane 120 is equal to a sum of a length of the upper tubular segment and a length of the lower tubular segment. Since the covering membrane 120 is a tubular structure with a certain strength, when applied to the inner/outer surface of the tubular main body 110, it can also enhance a strength of the tubular main body 110 and a connection strength between the upper tubular segment and the lower tubular segment. Simultaneously, it can maintain a preset form of the elastic curved segments, thereby preserving a shape of the tubular main body 110. The covering membrane 120 is usually made of polymer material, which can smooth an inner lumen of the tubular main body, prevent tissue hyperplasia of the atrial septum from blocking the tubular lumen, and maintain patency of the tubular lumen. The material of the covering membrane 120 may be a polymer material or a biological material, including but not limited to polyester, polytetrafluoroethylene, animal pericardium, or the like. A surface of the covering membrane 120 may be provided with a coating to improve smoothness of the surface, thereby enhancing long-term patency rates. In some alternative embodiments, the covering membrane 120 may also be disposed on the outer surface of the tubular main body 110. In other alternative embodiments, a layer of covering membrane 120 may be disposed on both the inner surface and outer surface of the tubular main body 110. Another beneficial effect of the covering membrane 120 is that an insulating function is provided, which may isolate the tubular main body 110 from the wireless sensor 200 to a certain extent, thus avoiding signal interference. The covering membrane 120 may be connected to the tubular main body 110 by means of heat pressing or suturing. In the figures, to more clearly distinguish the covering membrane 120 from the tubular main body 110, a thickness dimension of the covering membrane 120 is intentionally exaggerated and the covering membrane 120 is depicted on the inner surface of the tubular main body 110, whereas its actual thickness is smaller than illustrated.

It should be understood by a person skilled in the art that, although in the embodiment, the stent 100 and the wireless sensor 200 are connected via the connecting member 300, in other embodiments, the stent 100 may also be used as a standalone shunt.

In the embodiment, the engaging segment includes a first engaging segment and a second engaging segment, which are integrally formed with respective portions of two elastic curved segments. In other words, the first engaging segment is formed by natural extension of a portion of the elastic curved segment, and the second engaging segment is formed by natural extension of a portion of the other elastic curved segment. Please refer to FIG. 6, which is an enlarged schematic diagram of the junction between the upper tubular segment 10 and the lower tubular segment 20 with the sensor omitted. At a middle portion of the tubular main body 110, an initial segment (upper portion) of the elastic curved segment of the lower tubular segment 20 forms the first engaging segment 114, and a terminal segment (lower portion) of the elastic curved segment of the upper tubular segment 10 forms the second engaging segment 115.

With reference to to FIG. 2. The wireless sensor 200 includes a detection element 210 and a wireless signal transmission element 220. The detection element 210 may be a pressure detection element, which may measure, for example, a pressure of blood flow. The detection element 210 is disposed at one end of the wireless sensor 200 and is configured to monitor a pressure of the left atrium. The wireless signal transmission element 220 is configured to transmit signals collected by the detection element 210 to an outside of the human body in a form of electromagnetic waves. The detection element 210 and the wireless signal transmission element 220 are encapsulated as an integral unit by a sealed structure. In other embodiments, two detection elements 210 may also be disposed at two ends of the wireless sensor 200 to detect the pressure of the left atrium and the right atrium, with the wireless signal transmission element 220 disposed between the two detection elements 210. Preferably, the wireless sensor 200 further includes a housing. The detection element 210 is located at one end of the housing and protrudes a predetermined distance relative to the tubular main body 110 in a length direction of the housing. That is, at an end where the detection element 210 is located, a sensing surface of the wireless sensor 200 protrudes relative to the tubular main body 110 to prevent intimal hyperplasia on the sensing surface of the detection element 210.

In the embodiment, the connecting member 300 is an annular sleeve member. A local segment of the annular sleeve member protrudes slightly outward to form a second connecting portion 320, and a remaining portion other than the second connecting portion 320 constitutes a first connecting portion 310. An outward protrusion of the second connecting portion 320 may be formed when it is pulled by the stent 100 and the wireless sensor 200 after the connecting member 300 connects them, or it may be a permanent protrusion set during manufacturing. The first connecting portion 310 is wound around a middle portion of the wireless sensor 200. The second connecting portion 320 is a thin-sheet structure as a whole, including a sheet portion 321. The sheet portion 321 is approximately rectangular. The sheet portion 321 has two parallel edges, that is, an upper edge and a lower edge which are perpendicular to an axis of the annular sleeve member. The upper edge together with at least a portion of a region adjacent to the upper edge forms one edge portion 322, and the lower edge together with at least a portion of a region adjacent to the lower edge forms another edge portion 322. The two edge portions 322 are configured to be clamped by the first engaging segment 114 and the second engaging segment 115, that is, the two edge portions 322 form a clamped portion.

In this embodiment, a width of the annular sleeve member (a dimension in an axial direction) is relatively small, while lengths of the first engaging segment 114 and the second engaging segment 115 are relatively large. Therefore, both the first engaging segment 114 and the second engaging segment 115 clamp the two edge portions 322 respectively. That is, the first engaging segment 114 and the second engaging segment 115 clamp an entire axial region of the sheet portion 321. In some alternative embodiments, an axial dimension of the sheet portion 321 is relatively large, and/or the length of the first engaging segment 114 and/or the second engaging segment 115 is relatively small. The first engaging segment 114 may clamp only the edge portion 322 corresponding to the lower edge, while the second engaging segment 115 clamps only the edge portion 322 corresponding to the upper edge. Relative to an overall size of the connecting member, a protruding height of the second connecting portion 320 is very small. In some alternative embodiments, the first connecting portion may also be a complete tubular structure, and the second connecting portion 320 may be regarded as a structure attached to an outer contour of the first connecting portion 310, so that a radial dimension at a portion where the second connecting portion 320 and the first connecting portion 310 are attached does not increase significantly. The term "attached" referred to herein may mean that two adjacent surfaces are in contact with each other (but may be separated by an external force, such as being separated by insertion of an engaging segment), or that the two adjacent surfaces have a very small distance, thereby allowing insertion of the first engaging segment.

By means of the aforementioned connection method, the second connecting portion 320 of the connecting member 300 is clamped between the first engaging segment 114, the second engaging segment 115, and the covering membrane 120, which is equivalent to pressing the second connecting portion 320 against a surface of the tubular main body 110. The stent 100 and the wireless sensor 200 are connected together in parallel and in an externally tangent manner. Moreover, the first engaging segment 114 and the second engaging segment 115 are also clamped between the wireless sensor 200 and the second connecting portion 320. Such a connection is simple and reliable, facilitating an assembly and connection of minute components like the shunt. Moreover, because free ends of the first engaging segment 114 and the second engaging segment 115 point in opposite directions, axial dislodgment of the second connecting portion 320 is also prevented. In a connection method where the engaging segments on the surface of the stent 100 clamp the sheet structure on the surface of the wireless sensor 200, a dimension of a connection structure in a radial direction is very small, which allows the wireless sensor 200 to be closely connected to the stent 100 without causing an overall radial dimension of the implant to become excessive.

As long as the first connecting portion 310 can be fixedly connected to the wireless sensor 200 and the second connecting portion 320 can be connected to the first connecting portion and the engaging segment of the tubular main body 110, a shape of the connecting member 300 is not limited to the structure described in the foregoing embodiment. Therefore, the first connecting portion 310 may also be an annular structure with an opening, which may clamp around the wireless sensor 200; or a closed or unclosed box-like structure adapted to a shape of the wireless sensor 200, which may accommodate the wireless sensor 200; or a structure designed to match a portion of the wireless sensor 200 to be connected, such as one provided with snap-fits, threads, or adhesive surfaces (of course, the wireless sensor itself needs to be provided with corresponding mating structures such as slots, threads, or adhesive surfaces). The second connecting portion 320 may be an arched structure, a cantilever structure, or the like that can be clamped by the engaging segment.

Preferably, the connecting member 300 is a sleeve, a portion of which forms the first connecting portion, and another portion forms the second connecting portion. When the connecting member 300 and the wireless signal transmission element 220 at least partially overlap in the axial direction, the connecting member 300 is a non-metallic sleeve, and the non-metallic sleeve whose cross-section is a closed structure (for example, an O-shaped structure) or an unclosed structure (for example, a C-shaped structure). In this way, an interference from the connecting member 300 with signal transmission may be avoided. Alternatively, the annular sleeve member is a metallic sleeve, and a cross-section of the metallic sleeve is an unclosed structure, which can also prevent the connecting member 300 from affecting signal transmission. When the connecting member 300 and the wireless signal transmission element 220 do not overlap at all in the axial direction, there is no restriction on a material of the connecting member 300.

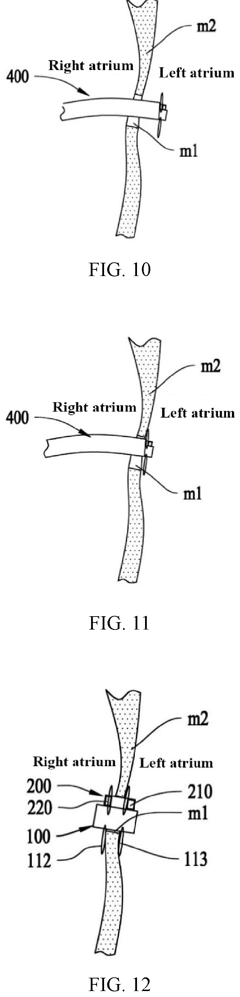

FIG. 12 shows an implant when installed at a target site. The target site m1 is an aperture connecting a left atrium and a right atrium. The implant is inserted as a whole into the aperture and is clamped onto atrial septum tissue by the first clamping arm 112 and the second clamping arm 113 to prevent the implant from axially dislodging from the target site m1. The detection element 210 is located in the left atrium and is capable of detecting a blood pressure of the left atrium. When the left atrium contracts, in addition to entering the left ventricle, a portion of blood flow also enters the right atrium through a lumen of the stent 100. The wireless sensor 200 is arranged side by side outside the stent 100, so the wireless sensor 200 does not obstruct the blood flow inside the lumen of the stent 100 or two ends of the stent 100. Furthermore, the stent 100 does not surround the wireless sensor 200, and a solid structure enclosing the opening 130 has no closed-loop structure in a circumferential direction within at least one axial region; thus, there is no obstruction to wireless signal transmission. The wireless sensor 200 can be installed side by side with the stent 100 in the atrial septum aperture without occupying excessive atrial space as would be a case with traditional axial serial arrangements. The connecting member 300 is tightly connected to the engaging segment of the stent 100, causing the stent 100 and the wireless sensor 200 to closely abut each other. This not only enhances a connection strength between the two but also reduces radial dimensions of the two, which is beneficial for transvascular interventional implantation procedures.

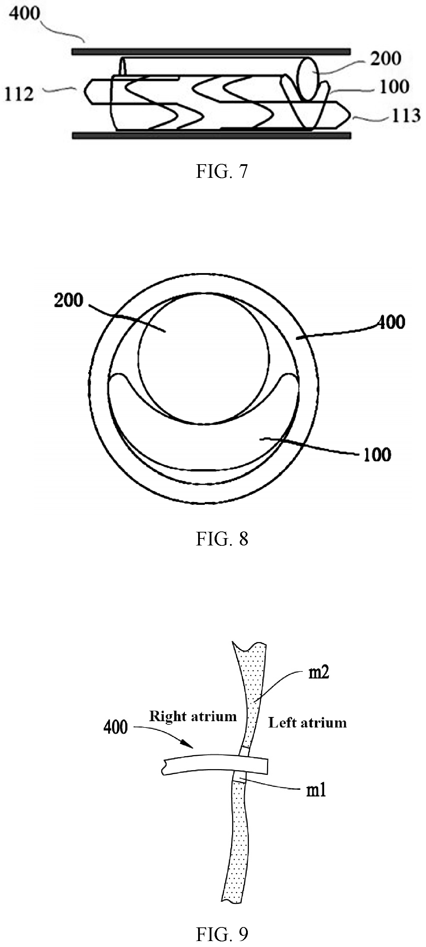

Referring to FIGS. 7 and 8. FIG. 7 is a schematic diagram of the implant according to the foregoing embodiment in a compressed state when located within a delivery catheter. FIG. 8 is a schematic diagram showing an axial profile projection plane of the delivery catheter and the implant in FIG. 7. The stent 100 of the implant is formed by an elastic curved segment, so an entire structure of the stent 100 may deform under external compressive force and restore its original shape upon force removal. The implant and the delivery catheter used for the interventional surgery constitute an implant system.

In the implant, the elastic curved segment forms the main body of the stent 100, imparting elasticity to the entire stent. When pressure is applied from the wireless sensor 200 toward the stent 100 along a line connecting centers of the wireless sensor 200 and the stent 100, the stent 100 may be compressed. That is, the stent 100 is elastically deformable along a first radial direction within a confined space (a radial dimension decreases in a direction of an applied force while increases in a direction which is perpendicular to the applied force); the "first radial direction" here refers to a radial direction aligned with the applied force). The wireless sensor 200 can partially embed into a recessed area of the stent 100. Once the external force is removed, the stent 100 will restore its original shape and positional relationship with the wireless sensor 200. During a process of placing the implant into the delivery catheter 400, a tube wall of the delivery catheter 400 exerts pressure on the stent 100 and the wireless sensor 200 both along a direciton of the line connecting centers and along a radial direction perpendicular to the direciton of the line connecting centers. This causes the stent 100 to be compressed radially into a state where its axial profile projection plane is C-shaped, while the wireless sensor 200 partially embeds into the recessed area of the stent 100. The "axial profile projection plane" here refers to a profile image obtained by projecting an object along an axial direction of the object. Furthermore, the first clamping arm 112 and the second clamping arm 113 are pressed flat along their longitudinal cross-sections to lie close to a surface of the stent 100. That is, a free end of the first clamping arm 112 and a free end of the second clamping arm 113 move toward the extension directions of the respective ends of the stent 100. After being placed into the delivery catheter 400, an overall radial dimension of the implant is compressed. Because the compressive deformation takes a form of flattening the tubular main body, a perimeter of the tubular main body remains unchanged. This reduction in radial dimension is not achieved by axial elongation, so the axial dimension of the implant does not change significantly. An implant with a shorter length is more conducive to a bending of the delivery catheter 400 and improves convenience of the implantation procedure.

Referring to FIGS. 9 to 12, which illustrate a surgical procedure for implanting a shunt at a target site via an interventional catheter. FIG. 9 is a schematic diagram showing the delivery catheter 400 containing the implant upon reaching the target site m1. FIG. 10 is a schematic diagram showing the implant with one end and the first clamping arm 112 protruding from the delivery catheter 400. FIG. 11 is a schematic diagram showing the first clamping arm 112 of the implant reaching a surface of the atrial septum m2 upon retraction of the delivery catheter 400. FIG. 12 is a schematic diagram showing the implant being installed at the target site m1 after withdrawal of the delivery catheter 400.

One end of the delivery catheter 400 containing the implant enters a right atrium through a relatively large blood vessel in a human body and proceeds into a left atrium through an aperture in the atrial septum m2. At this position, one end of the implant is extended from an end of the delivery catheter 400 by either retracting the delivery catheter or pushing the implant outward, and the first clamping arm 112 is expanded in a cantilever state. After the first clamping arm 112 is expanded, an overall radial dimension of the implant is larger than a diameter of the aperture in the atrial septum m2. The delivery catheter is then withdrawn further, and the implant moves with the tube until the first clamping arm 112 contacts the atrial septum m2. Upon continued withdrawal of the delivery catheter 400, the implant remains at this position without retreating with the delivery catheter 400 due to obstruction by the atrial septum m2. After the implant completely detaches from the delivery catheter 400, the second clamping arm 113 also expands. Thereby, the second clamping arm 113 and the first clamping arm 112 retain the stent 100 and the wireless sensor 200 within the aperture of the atrial septum m2. Furthermore, the stent 100 restores to its original shape in a radial direction, and a lumen of the stent 100 allows blood flow from the left atrium to enter the right atrium. Once the stent 100 is in place, the detection element 210 of the wireless sensor 200 may monitor a blood pressure of the left atrium in real time and transmit relevant information to an external receiving device via the wireless signal transmission element 220.

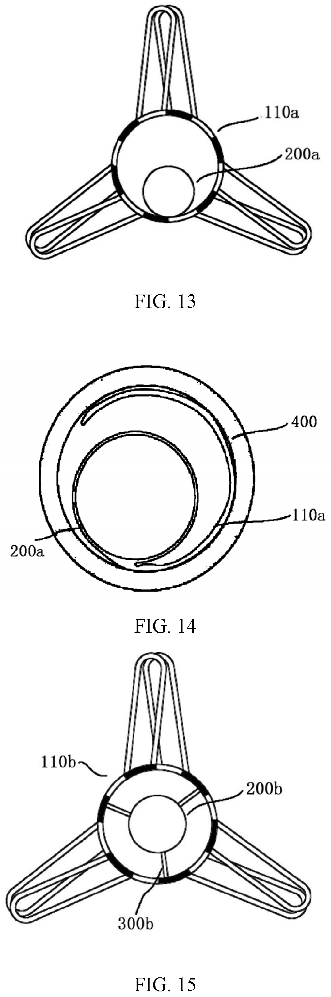

Referring to FIGS. 13 and 14. FIG. 13 is a schematic structural diagram of an implant according to another embodiment of the present invention. FIG. 14 is a schematic structural diagram of an axial profile projection plane of the implant in FIG. 13 when located within a delivery catheter. In the embodiment, a tubular main body 110a of the implant has a same structure as the tubular main body 110 in the foregoing embodiment, and a wireless sensor 200a is located inside the tubular main body 110a and is internally tangent to the tubular main body 110a. When the implant is compressed within the delivery catheter 400, a portion of the tubular main body 110a completely envelops the wireless sensor 200a, while a remaining portion of the tubular main body 110a is folded to surround an outer side of the wireless sensor 200a.

It should be understood that although FIGS. 2 to 6 use a configuration with the wireless sensor 200 placed outside the tubular main body 110 as an example, the connecting member and engaging segments shown in FIGS. 2 to 6 may also be used to fix the wireless sensor 200 to the tubular main body 110 when the wireless sensor 200 is placed inside the tubular main body 110. The covering membrane 120 can be disposed on the outer side of the tubular main body 110 or simultaneously on both the inner and outer sides of the tubular main body 110. When a portion of the covering membrane 120 is disposed on the inner side of the tubular main body 110, the engaging segment may pass through the covering membrane 120 to connect with the connecting member.

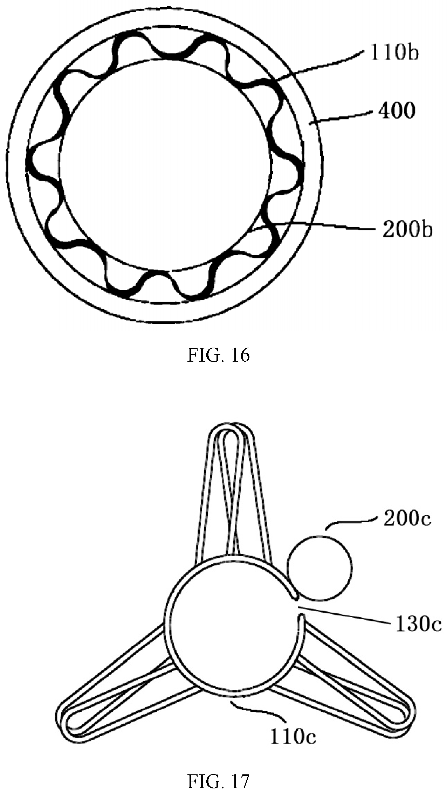

Referring to FIGS. 15 and 16. FIG. 15 is a schematic structural diagram of an implant according to another embodiment of the present invention. FIG. 16 is a schematic structural diagram of an axial profile projection plane of the implant in FIG. 15 when located within a delivery catheter. In this embodiment, a tubular main body 110b of the implant has a same structure as the tubular main body 110 in the foregoing embodiment, and a wireless sensor 200b is located inside the tubular main body 110b and arranged coaxially with the tubular main body 110b. The wireless sensor 200b and the tubular main body 110b are connected by multiple limiting wires 300b. The limiting wires 300b may be made of a metallic material or may be made of a polymer material. The limiting wires 300b may be combined with the tubular main body 110b by means of knotting, bonding, spot welding, or the like. The limiting wires 300b may also be combined with the pressure monitoring device 200b in the foregoing ways, but combination methods are not limited to the examples given. When the implant is compressed within the delivery catheter 400, the tubular main body 110b is squeezed between the delivery catheter 400 and the wireless sensor 200b, forming a structure whose axial profile projection plane exhibits a wavy annular shape.

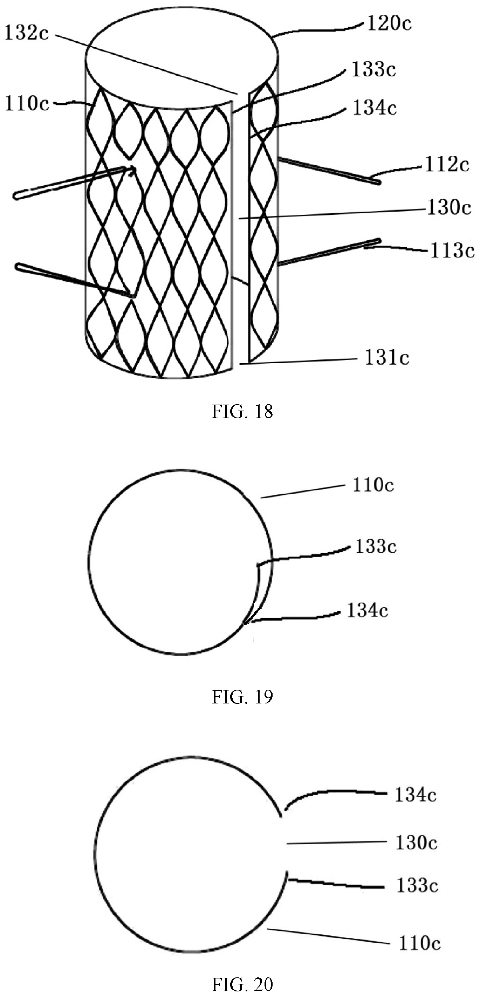

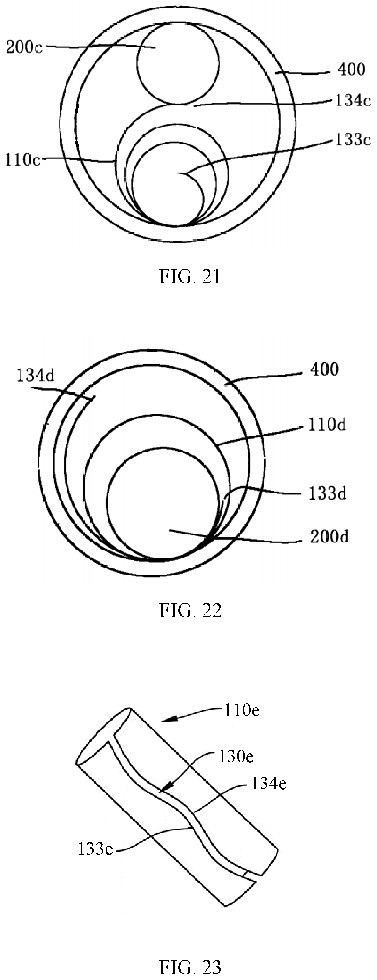

Referring to FIGS. 17 to 21. FIG. 17 is a schematic structural diagram of an implant according to another embodiment of the present invention. FIG. 18 is a schematic structural diagram of a stent of the implant in FIG. 17 with a wireless sensor omitted. FIG. 19 is a schematic structural diagram of an axial profile projection plane of the stent of FIG. 18 in a first state. FIG. 20 is a schematic structural diagram of an axial profile projection plane of the stent of FIG. 18 in a second state. FIG. 21 is a schematic structural diagram of an axial profile projection plane of the implant in FIG. 17 when located within a delivery catheter.

In this embodiment, the stent of the implant includes a tubular main body 110c, a first clamping arm 112c, and a second clamping arm 113c. The tubular main body 110c is an open-loop tubular structure formed by winding a mesh sheet woven from metal wires, exhibiting a C-shaped axial profile projection plane. The mesh sheet is rectangular when in an expanded state, with two opposite edge portions of the mesh sheet being a first end portion 133c and a second end portion 134c. The first end portion 133c and the second end portion 134c of the mesh sheet are not joined together, thereby forming an opening 130c between them. Two ends of the opening 130c are a first open end 131c and a second open end 132c. That is, the opening 130c may be regarded as a straight-line gap that penetrates a circumferential surface of the tubular main body 110c in a direction parallel to an axial direction.

When the tubular main body 110c is compressed, the first end portion 133c may be wound from an inner side of the second end portion 134c into an interior of the tubular body, thereby enabling the tubular main body 110c to have a smaller compressed volume. A diameter of the tubular main body 110c in a working state may be adjusted according to a size of the target site m1. For example, taking the target site as an aperture of an atrial septum: when the aperture is relatively small, the diameter of the tubular main body 110c may also be expanded or self-expanded to a size of the aperture, as shown in FIG. 19, in this case, the first end portion 133c and the second end portion 134c are offset from each other radially, and the second end portion 134c overlaps other portions of the tubular main body 110c. Under these circumstances, the covering membrane 120 is disposed on both an inner and outer sides of the tubular main body 110c to insulate the second end portion 134c from the other portions of the tubular main body 110c. When the aperture is relatively large, the diameter of the tubular main body 110c may also be expanded or self-expanded to the size of the aperture, as shown in FIG. 20, in this case, the first end portion and the second end portion are circumferentially offset from each other with a certain distance between them. When implementing this embodiment by using a balloon-expandable structure, both the tubular main body 110c and a tissue of the target site m1 may be simultaneously dilatedto a desired diameter via balloon post-dilatation. When implementing this embodiment by using a self-expanding structure, the tissue at the target site may be pre-dilated with a balloon, after which the tubular main body 110c is placed and allowed to expand to an intended size.

As shown in FIG. 21, during a compression process, the mesh sheet may be wound and compressed in a scroll-like manner. That is, the first end portion 133c of the mesh sheet enters the tubular main body from the inner side of the second end portion 134c and winds several turns around a center. In this case, the wireless sensor 200c is located outside the wound tubular main body 110c, and the wireless sensor 200c and the wound tubular main body 110c are arranged side by side within the delivery catheter 400.

Referring to FIG. 22, FIG. 22 is a schematic structural diagram of an axial profile projection plane of an implant when located within a delivery catheter in an optional embodiment. In this embodiment, a wireless sensor 200d is located inside a tubular main body 110d of a stent, and the two are in an internally tangent relationship. The tubular main body 110d has a same structure as the tubular main body 110c shown in FIG. 17. When the implant is compressed, a mesh sheet may be wound and compressed in a scroll-like manner. That is, a first end portion 133d of the mesh sheet enters the tubular main body from an inner side of the second end portion 134d and winds several turns around a center. Moreover, the wireless sensor 200d is wound inside the tube, resulting in the wound tubular body and the wireless sensor 200d being arranged substantially coaxially.

Referring to FIG. 23, FIG. 23 is a schematic structural diagram of a tubular main body of an implant according to another embodiment of the present invention. In this embodiment, a tubular main body 110e is a tubular body formed by winding a mesh sheet woven from metal wires. The tubular body is an open-loop tubular structure having a C-shaped axial profile projection plane. Furthermore, a first end portion 133e and a second end portion 134e of the mesh sheet are not engaged with each other, and an opening 130e exists between the two. Edges of the first end portion 133e and the second end portion 134e are curved, therefore the opening 130e is a wavy-shaped gap penetrating a circumferential surface of the tubular main body 110e in an axial direction.

It should be understood that in other embodiments, the opening 130c may also not be parallel to the axial direction of the tubular main body 110c. For example, in an expanded state, the mesh sheet may be a parallelogram rather than a rectangle. In other embodiments, the tubular main body 110c may be directly woven from metal wires by means of a weaving process, or directly cut from a metal tube by means of a cutting process, with an opening pre-reserved. The opening extends along a wall of the tubular main body 110c but does not penetrate the tubular main body 110c. As long as a sensor is placed in an area where the opening is located, a signal interference from the tubular main body 110c with the sensor may also be reduced.

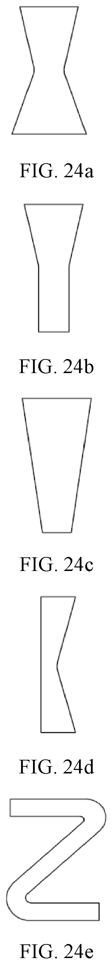

In the foregoing embodiments, the tubular main body has a cylindrical structure. In other embodiments, the tubular main body may also have other structures. Referring to FIGS. 24a to 24e, FIGS. 24a to 24e illustrate some optional implementations of a tubular main body of a stent in this embodiment.

FIG. 24a is a schematic diagram of a shape of a longitudinal profile projection plane of a stent in an optional implementation. In this implementation, a diameter of a middle portion of the stent is smaller than diameters at two ends of the stent, forming an X-shaped projection plane. FIG. 24b is a schematic diagram of a shape of a longitudinal profile projection plane of a stent in another optional implementation. In this implementation, an upper segment of the stent is trumpet-shaped and a lower segment is straight-tubular, forming a Y-shaped projection plane. FIG. 24c is a schematic diagram of a shape of a longitudinal profile projection plane of a stent in another optional implementation. In this implementation, a diameter of one end of the stent is larger than that at the other end, and a diameter of the tubular main body changes linearly along an axial direction, forming a V-shaped projection plane. FIG. 24d is a schematic diagram of a shape of a longitudinal profile projection plane of a stent in another optional implementation. In this implementation, diameters at two ends of the stent differ from that at a middle portion of the stent, and centers of the two ends are not coaxial with a center of the middle portion, so that the stent has a K-shaped projection surface. FIG. 24e is a schematic diagram of a shape of a longitudinal profile projection plane of a stent in another optional implementation. In this implementation, the stent as a whole is Z-shaped. The "longitudinal profile projection plane" refers to a profile image obtained by projecting an object along a longitudinal direction of the object.

It should be understood that in the several embodiments described above, the stent may also be separated from the sensor as an independent implant. Since there is an opening in the stent itself, no closed-loop metallic ring structure is formed in at least a portion of the region. Therefore, whether the sensor or other electronic device is implanted before, after, or simultaneously with the stent in a vicinity of the region, an interference from the stent with the wireless signal can be avoided or reduced. In a preferred solution, the opening is distributed over an entire length of the tubular main body, which may further increase flexibility in a relative positioning of the sensor or other electronic devices with respect to the stent and improve effect of reducing signal interference. Moreover, when the opening divides the tubular main body into an open-loop structure with a C-shaped axial profile projection plane, the diameter of the tubular main body may also be adaptively adjusted according to a size of the aperture at the target site, thereby solving a problem in the prior art that a mismatch between a size of the shunt and the target site leads to complex surgical procedures or suboptimal surgical outcomes.

The present invention also provides an implant system. The implant system includes a delivery catheter and an implant. The delivery catheter is configured to accommodate the implant in a compressed state and deliver the implant to a target site via artificial or natural cavities/vessels. The implant may include a stent and a wireless sensor. The stent may be the shunt for atrial shunt in the foregoing embodiments, or may be an occluder or a vascular stent. The implant is in a compressed state within the delivery catheter. For details of the compressed states, refer to the illustrations and descriptions in the foregoing embodiments showing the implant positioned inside delivery catheter. When the delivery catheter delivers the implant to the target site, the implant may be pushed out of the catheter by using an inner catheter.

In some optional embodiments, the implant system further includes a signal receiving device. The signal receiving device is configured to receive signals from the wireless sensor in the implant, allowing medical personnel to monitor changes of physiological parameters of a living body in real time.

The foregoing descriptions are merely specific implementations of the present invention. but the scope of protection of the present invention is not limited thereto. Any person skilled in the art can readily conceive of changes or substitutions within the technical scope disclosed in the present invention, and these should be covered within the protection scope of the present invention. Therefore, the protection scope of the present invention shall be subject to the protection scope of the claims.

## Claims

1. An implant for implanting at a target site within a living body, comprising:
a stent, configured to be disposed at the target site to improve a physiological function of the living body, wherein the stent comprises a tubular main body made of a metallic material, a circumferential surface of the tubular main body is provided with an opening, and the opening extends over at least a portion of a length of the tubular main body.

2. The implant according to claim 1, wherein the opening comprises at least a first open end.

3. The implant according to claim 2, wherein the opening is distributed over an entire length of the tubular main body and further has a second open end.

4. The implant according to any one of claims 1 to 3, wherein the opening extends on the circumferential surface of the tubular main body in a helical, linear, or wavy form.

5. The implant according to claim 4, wherein when the opening extends on the circumferential surface of the tubular main body in a helical form, the opening extends for at least two turns around the circumferential surface of the tubular main body.

6. The implant according to claim 5, wherein an elastic curved segment is helically coiled to form the tubular main body, and a gap between two adjacent turns of the elastic curved segment forms the opening.

7. The implant according to claim 6, wherein the elastic curved segment is a metal wire or a metal strip cut from a metal member.

8. The implant according to claim 6 or 7, wherein the elastic curved segment, starting from an initial position, extends circumferentially in an annular form for one turn, then extends helically upward for one or more turns, and then further extends in an annular form for one turn, thereby forming the tubular main body.

9. The implant according to claim 8, wherein the elastic curved segment is a wave shape.

10. The implant according to claim 4, wherein the opening divides the tubular main body into an open-loop structure having a C-shaped axial profile projection plane, and the open-loop structure has a first end portion and a second end portion opposite to each other.

11. The implant according to claim 10, wherein in a working state, the first end portion is not in contact with the second end portion, or is offset from the second end portion circumferentially or radially.

12. The implant according to claim 11, wherein the tubular main body has a mesh structure, and the mesh structure is formed by weaving metal wires or by cutting a metal member.

13. The implant according to any one of claims 10 to 12, wherein when the open-loop structure is compressed, a layered structure having a helical axial profile projection plane is formed, and the first end portion is wound from an inner side of the second end portion toward a center.

14. The implant according to any one of claims 1 to 13, further comprising a wireless sensor, wherein the wireless sensor comprises a detection element and a wireless signal transmission element, the detection element is configured to obtain a target physiological parameter, and the wireless signal transmission element is configured to transmit the target physiological parameter obtained by the detection element to an outside of the living body.

15. The implant according to claim 14, wherein in a working state, the wireless sensor is located outside the tubular main body, and when the tubular main body is compressed, the wireless sensor is located side by side outside the tubular main body.

16. The implant according to claim 14, wherein in a working state, the wireless sensor is located inside the tubular main body, and when the tubular main body is compressed, the wireless sensor is located inside the tubular main body.

17. The implant according to any one of claims 14 to 16, wherein the wireless sensor further comprises a housing, and the detection element is located at one end of the housing and protrudes a predetermined distance relative to the tubular main body along a length direction of the housing.

18. The implant according to any one of claims 1 to 17, wherein the stent further comprises a clamping arm assembly, the clamping arm assembly at least comprises a first clamping arm and a second clamping arm, the first clamping arm and the second clamping arm are distributed on an outer periphery of the tubular main body at intervals along an axial direction of the tubular main body, and the first clamping arm and the second clamping arm each forms a cantilever protruding from the outer periphery of the tubular main body.

19. The implant according to claim 18, wherein the first clamping arm and/or the second clamping arm are formed by bending an elastic metal wire or metal strip.

20. The implant according to claim 18 or 19, wherein the first clamping arm and/or the second clamping arm have a U-shaped or V-shaped structure formed by an elastic metal wire or metal strip extending radially a predetermined distance and then folding back.

21. The implant according to any one of claims 18 to 20, wherein the first clamping arm and/or the second clamping arm are integrally formed with the tubular main body.

22. The implant according to any one of claims 1 to 21, wherein the stent further comprises a covering membrane, the covering membrane is applied to an inner side and/or an outer side of the tubular main body, and the covering membrane is made of an insulating material.

23. The implant according to any one of claims 1 to 21, wherein the implant further comprises a wireless sensor and a connecting member, the stent and the wireless sensor are arranged in parallel, the wireless sensor is connected to an engaging segment of the stent via the connecting member, and the engaging segment is integrally formed with the tubular main body.

24. The implant according to claim 23, wherein the engaging segment comprises a first engaging segment and a second engaging segment, the connecting member comprises a first connecting portion and a second connecting portion, the first connecting portion is connected to the wireless sensor, the second connecting portion comprises a sheet portion, and the sheet portion is clamped by the first engaging segment and the second engaging segment.

25. The implant according to claim 24, wherein a free end of the first engaging segment and a free end of the second engaging segment point in opposite directions.

26. An implant system, comprising a delivery catheter and an implant, wherein the implant is the implant according to any one of claims 1 to 25, the implant is in a compressed state when disposed within the catheter, and the implant is capable of protruding from the catheter.

27. The implant system according to claim 26, wherein the tubular main body is compressed into a structure having an axial profile projection plane that is C-shaped, helical, or wavy closed-loop.

28. The implant system according to claim 27, wherein when the tubular main body is compressed into the structure having the axial profile projection plane that is C-shaped, an outer surface of a compressed tubular main body at least partially surrounds the wireless sensor.

29. The implant system according to claim 27, wherein when the tubular main body is compressed into the structure having the axial profile projection plane that is helical, the wireless sensor is located side by side outside the tubular main body, or the wireless sensor is located inside the tubular main body.

30. The implant system according to claim 27, wherein when the tubular main body is compressed into the structure having the axial profile projection plane that is wavy closed-loop, the wireless sensor is located inside the tubular main body.

31. The implant system according to any one of claims 26 to 30, further comprising a signal receiving device, wherein the signal receiving device is configured to transmit signals with the wireless sensor in the implant.
